# EUROPEAN PATENT SPECIFICATION

(11) **EP 0 255 974 B1**
(45) Date of publication and mention of the grant of the patent: **16.06.1993**
(21) Application number: 87201433.7
(22) Date of filing: 24.07.1987
(51) Int. Cl.: A61B 17/36, A61N 5/06

(54) **Apparatus for light coagulation of the morbid part**
Vorrichtung für Lichtkoagulation des morbiden Teiles
Dispositif de photocoagulation de tissu morbide

(30) Priority: 25.07.1986 JP 176265/86; 05.03.1987 JP 51748/87
(43) Date of publication of application: 17.02.1988
(73) Proprietor: Kinki University, Higashiosaka-shi Osaka (JP); Showa Aluminum Kabushiki Kaisha, Sakaishi Osaka (JP)
(72) Inventor: Tsujimura, Daijiro, Tondabayashi-shi Osaka (JP); Kagen, Hideki, Tondabayashi-shi Osaka (JP); Tsukamoto, Kenji, Sakai-shi Osaka (JP); Kikuchi, Shigeyuki, Sakai-shi Osaka (JP)
(74) Representative: Smulders, Theodorus A.H.J., Ir.

(56) References cited:
- EP-A- 0 246 127
- WO-A-85/05263
- AT-B- 380 634
- DE-A- 2 106 470
- US-A- 3 821 510

## Description

This invention relates to an apparatus for treatment of portions of a human body with laser light, comprising an optical fiber and a laser light source for generating laser light and for leading the light to the optical fiber, a flexible barrel in which the optical fibre is inserted such that a space exists between the outside of the optical fibre and the inside of the barrel, means for supplying cooling liquid into the space between the outside of the optical fibre and the inside of the barrel, an end portion of the optical fibre being positioned inner to an end portion of the barrel in axial alignment therewith, the end portion of the barrel comprising a cylindrical portion made of metal.

An apparatus of this type is disclosed in AT-B-0,380,634. The known apparatus is an apparatus in which shock waves are being produced by means of an optical breakdown. With said shock waves parts of a human body can be treated. The cylindrical end portion of the barrel of the known apparatus comprises a metallic cone, which serves both as a concentrator of the laser light and as a protector of the fibre end surface.

In case the apparatus for treatment of portions of a human body with laser light is an apparatus for light coagulation of a morbid part, it would be advantageous to have an end portion of the barrel that is exchangeable. In an apparatus for light coagulation of a morbid part, which, for instance, for external use is disclosed in US-A-3,821,510, the end portion of the barrel may contact the morbid part to be treated and may easily be damaged due to such contacts and due to local overheating by the laser light. Therefore, the end portion should be exchangeable. In case the end portion is exchangeable and replaceable, care should be taken not to damage the end portion of the optical fibre when exchanging the end portion of the barrel.

The invention now has a purpose to provide for an apparatus for light coagulation of a morbid part in which the end portion of the barrel is exchangeable and replaceable and of which the construction is such that the replacement of such end portion can be performed easily without the risk of damaging the end face of the optical fibre.

In accomplishing the above object, the invention provides an apparatus for light coagulation of a morbid part, in which the end portion of the barrel (21) comprises a cylindrical metal connecting member (23) having a connecting portion (26) and a flange part (27), the connecting portion (26) being cut such as to taper so as to comprise portions of truncated cones each of said portions having a larger diameter at the side of the flange part (27) of the connecting member (23), the connecting portion (26) being inserted into the outer end of the barrel (21), the flange part (27) being adjacent to the connecting portion (26) in axial direction and projecting in radial direction and contacting an end face (21a) of the barrel (21), a threaded part (28) at the side of the flange part (27) opposite to the connecting part (26), and a cylindrical metal contact member (24,49,52) being threaded (25) such as to being coupled with the threaded part (28) of the connecting member (23), the end portion (29a) of the optical fibre (29) extending into, but not beyond the connecting member (23).

In a preferred embodiment the cylindrical metal contact member is trumpet-shaped, the part having the larger diameter being positioned at the end side.

In the apparatus according to the invention the cylindrical metal contact member preferably is made of aluminium alloy.

In the apparatus according to the invention the transparant cooling water is in contact with the end face where the laser light of the optical fiber is illuminated. Therefore, the optical fiber is sufficiently cooled herewith, the distribution of the laser light is made uniform and the life of the optical fiber is lengthened. Additionally, laser light of a high output can be used to illuminate the morbid part.

The end portion of the optical fiber is positioned inner to the end portion of the barrel in axial alignment therewith so that the end portion of the barrel can make contact with the surface of the tissue to be coagulated with light and the light coagulation of the desired area can be carried out accurately. It is also possible to illuminate the laser light from a tangential direction to the morbid part to carry out the light coagulation.

The end portion of the optical fiber is positioned inner in the axial alignment in the state that the end of the barrel is in contact with the morbid part and therefore, the end portion of the optical fiber is never in contact with the morbid part. Accordingly, the end portion of the optical fiber is prevented from adhesion of tissues, blood and the like. Generation of heat to an abnormal high temperature is also prevented. Therefore, the optical fiber is protected from damage.
The cooling water is transparent and the laser light is naturally not intercepted.

According to this invention, the distribution of luminous intensity of the laser light illuminated to the morbid part is uniform and a local generation of an area heated to a high temperature is prevented so that the life of the optical fiber is extended and a treatment can be carried out with laser light of a high output and also at a low cost.

Furthermore, the end portion of the barrel is made of metal, for example, aluminum alloy and the inside has a specific luster depending on the metal. The reflection ratio is improved. Accordingly, the laser light from the optical fiber can be efficiently reflected and led to the morbid part. The end portion of the barrel is exchangeable. It will be exchanged by itself when the end part is damaged, worn away or stained. Thus, the maintenance is easy and other parts having a low possibility of damage may be used continuously for a long period.

These and other objects, features and advantages of the invention will become more apparent upon a reading of the following detailed specification and drawing, in which:
Fig. 1 is a cross-sectional view of a prior art,
Fig. 2 is a cross-sectional view of other prior art,
Fig. 3 is a cross-sectional view of an example of this invention,
Fig. 4 is a disassemble perspective side view of a connecting member 23 and the surroundings shown in Fig. 3,
Fig. 5 is a cross-sectional view of a part of an endoscope,
Fig. 6 is a cross-sectional view of Fig. 5 observed from the line VI - VI,
Fig. 7 is a cross-sectional view of other example of this invention,
Fig. 8 is a cross-sectional view of other example of this invention, and
Fig. 9 is a cross-sectional view of other example of this invention.

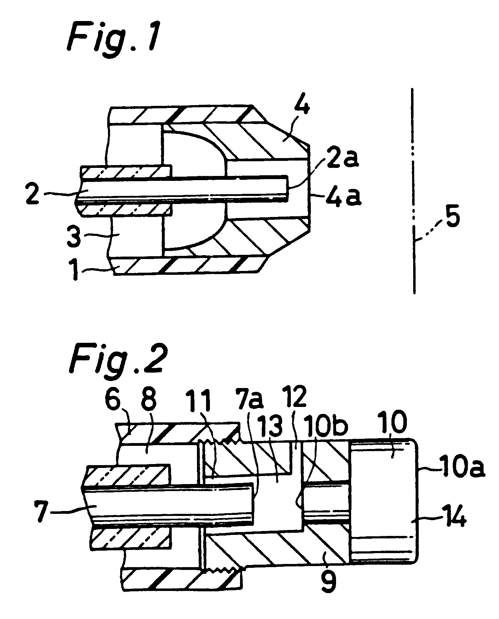

A typical prior art is shown in Fig. 1. An optical fiber 2 is inserted through a barrel 1 composed of, e.g., polyethylene and the space 3 between them is supplied with carbonic acid gas for washing and cooling the end face 2a of the optical fiber 2. The end portion of the barrel 1 has a protective member 4 composed of metal. The end face 2a of the optical fiber 2 is located inner to the end face 4a of the protective member 4, e.g., less than about 1 mm, in the axial alignment (left side of Fig. 1) and as the result the end face 2a of the optical fiber 2 is protected from damage when the barrel 1 and the optical fiber 2 are pushed into and moved in the axial direction. Laser light is led through the optical fiber 2. Light coagulation of a morbid part is carried out by illuminating laser light in the condition that the space between the surface of the morbid part 5 and the end face 2a of the optical fiber 2 positioned at the end portion of the barrel 1 is made to be, for example, about 1-2 cm so that the end portion of the optical fiber 2 is prevented from damaging when tissues adhere around the end face 2a of the core composed of quartz glass and become to an abnormal high temperature.

The space between the surface of the morbid part 5 and the end face 2a of the optical fiber 2 is varied by breathing of a man or the peristalisis of the stomach when the morbid part 5 is the wall of the stomach in the prior art shown in Fig. 1. Therefore, it is difficult to carry out the light coagulation of the tissues of the morbid part 5 in the desired condition. Additionally, when the tissues, blood and others adhere to the end face 2a of the optical fiber 2, they are heated to be at a high temperature and damage the end portion of the optical fiber 2.

Furthermore, there is a problem that laser light of a high output, e.g., about 70 - 90 W should be led into the optical fiber 2 for light coagulation at a desired strength since a space exists between the end face 2a of the optical fiber 2 and the morbid part 5.

Additionally, the carbonic acid gas supplied to the space 3 for washing and cooling is comparatively large quantity of flow, e.g., 400cc/minute or more. Therefore, the stomach of the patient is swollen in a comparatively short time when the barrel 1 was inserted into the stomach, which gives pains to the patient and there is the possibility that the tissue, e.g. wall of a digestive tract will be bored erroneously with the pressure of the gas.

Another prior art as known from WO-A-8505263 for dissolving such a problem is shown in Fig. 2. An optical fiber 7 is inserted through the barrel 6 and the space 8 is fed with cooling water by a compressor. The end portion of thee barrel 6 has a connecting member 9 composed of metal and a contact member 10 composed of new ceramic, e.g., artificial sapphire connected to the connecting member 9. The end face 10a of the contact member 10 is an plane being vertical to the axis extending to right and left in Fig. 2. A passage 11 is formed between the connecting member 9 and the optical fiber 7. In the connecting member 9, water passed through the passage 11 and fed from the space 8 passes through the space 13 between the end face 7a of the core layer of the optical fiber 7 and the end face 10b of the contact member 10 facing to the optical fiber 7 of the contact member 10, and is supplied to wash and cool the end face 7a of the core layer of the optical fiber 7 and the end face 10b of the contact member 10 facing to the optical fiber 7, and a jet of water is sent out from the nozzle 12 to wash and cool the contact member 10. In the prior art shown in Fig. 2, the light coagulation is carried out by contacting the end face 10a of the contact member 10 to the tissue to be coagulated optically so that the morbid part can be coagulated optically in the desired state even if the morbid part of the tissue to be coagulated optically is the wall of the stomach and it stirs with vermiculation and the like and furthermore the morbid coagulation may be carried out by illumination of laser light from the tangential direction.

This invention has a benefit that the laser light supplied to the optical fiber 7 may have lower output, e.g., about 10 - 20 W compared with the prior art noted in Fig. 1, since the light coagulation is carried out by contacting the contact member 10 composed of new ceramic such as artificial sapphire having a high melting point to the morbid part.

The prior art shown in Fig. 2 has a new problem. Namely, the laser light refracts and concentrates into the position around the center shown by the reference symbol 14 at the end face 10a of the contact member 10 and the position and its around are heated to higher temperature compared with the part around the heated part and a uniform distribution of the laser light cannot be obtained over the whole of the end face 10a. Accordingly, uniform light coagulation of the tissue cannot be resulted in the morbid part contacting to the end face 10a of the contact member 10. The other problem of the prior art shown in Fig. 2 is that the position 14 around the center of the contact member 10 may become depressed and easily damaged since the laser light concentrates to the position and heats it to a high temperature. Accordingly, it is difficult to conjecture the coagulating state of the morbid part during the treatment with the laser light illumination. Additionally, the life of the contact member 10 is short.

The light coagulation with laser light of a high output cannot be carried out since heat is generated locally at the position 14 around the center of the contact member 10. The contact member 10 is composed of new ceramic such as artificial sapphire as noted above and therefore it is expensive.

Referring now to the drawings, embodiments of the invention are described below.

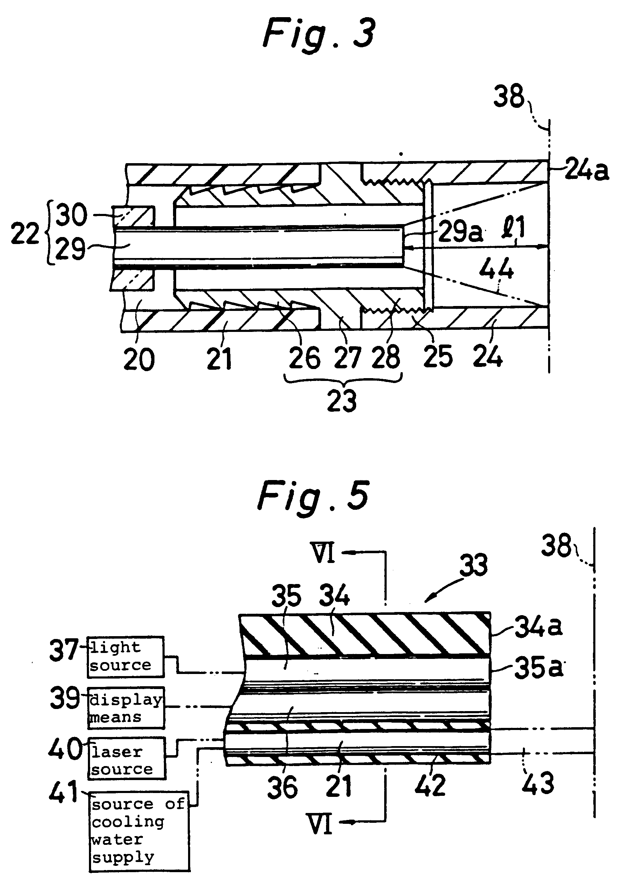

Fig. 3 is a cross-sectional view of an example of this invention. An optical fiber 22 having flexibility is inserted into a barrel 21 composed of a flexible material, e.g., polyethylene. The end portion of the barrel 21 is composed with a connecting member 23 made of, e.g., stainless steel and a contact member 24. The contact member 24 is composed of aluminum alloy with high hardness but is endurable to fine threading work and the like. The contact member 24 may be composed of other metals such as stainless steel and the like. The contact member 24 is a right cylinder of which inside is produced to have glaze so as to reflect laser light with a high efficiency and is, e.g., 1.4 mmφ-2 mmφ. The wall thickness is, e.g. 0.2 - 0.4 mm. A female thread is cut on the inner periphery surface of the starting portion of the contact member 24. The connecting member 23 has a connecting portion 26 which may be inserted into the barrel 21 composed of polyethylene and the like, a flange portion 27 and a threading portion 28 having a male thread. The male thread 25 of the contact member 24 is freely screwed in and out with the threading portion 28. The connecting portion 26 is cut to taper so as to retain the portions of the truncated cones having the largest diameter towards the flange portion 27 in axial alignment so that it is inserted into the barrel 21 easily and prevented from slipping out.

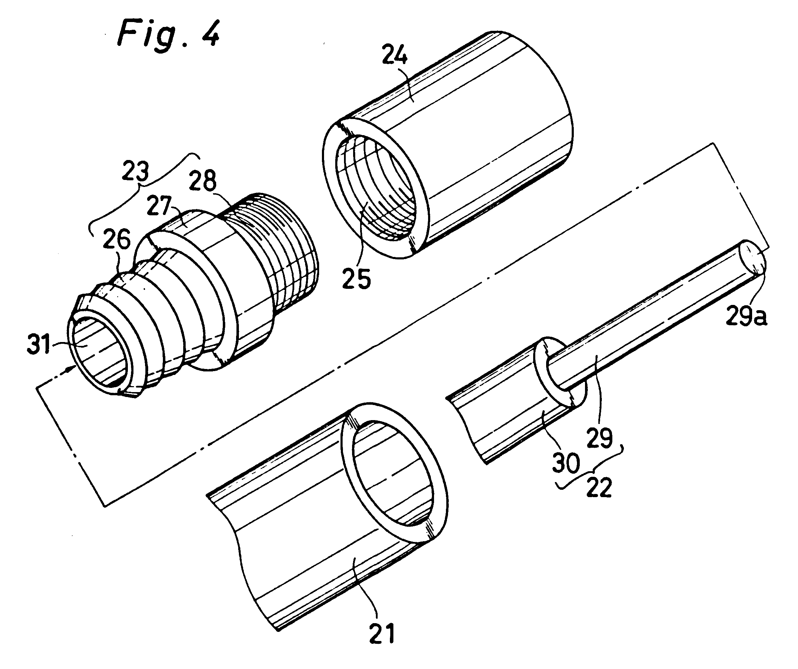

Fig. 4 shows a disassemble perspective side view of the connecting member 23 and the surroundings. The optical fiber 22 is provided with a core layer 29 and a clad layer 30 surrounding the core layer 29. The clad layer 30 is removed near at the end portion of the optical fiber 22 and the resultant bare core layer 29 is inserted into a through hole 31 of the connecting member 23. The end face 29a of the core layer 29 is at the inner position sunken from the end face 24a of the contact member 24 (the left side in Fig. 3) at the distance of ℓ1 in axial alignment. The distance of ℓ1 is, e.g., about 5 mm.

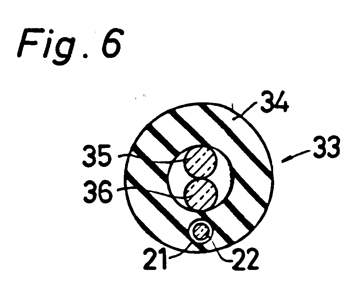

Fig. 5 is a cross-sectional view of an endoscope 33 carried out in relation to the present invention.

Fig. 6 is a cross-sectional view from the line VI-VI. In the body 34 of the endoscope 33 there are equipped with an optical fiber for lighting 35 and an optical fiber for image transmission 36. A light source 37 is connected to the optical fiber for lighting 35 and the light from the light source 37 reaches to the morbid part 38 to be coagulated with light and the surroundings through the optical fiber for lighting 35 and the end face 35a. The image of the lighted portion of the morbid part 38 is transmitted to display means 39 through the optical fiber for image transmission 36. The display means 39 may be composed of the one for direct ocular observation having an eyepiece or may be composed of the one displaying with means such as a cathode ray tube.

Laser light is led from a laser source 40 to the optical fiber 22 deposited in the barrel 21 of the apparatus for light coagulation of this invention. The laser source 40 is, e.g., Nd-YAG laser and the output is, e.g. 20 - 30 W. The barrel 21 is also connected with a source of cooling water supply 41. From the source of cooling water supply 41, cooling water is made pass through the space 20 between the outside of the optical fiber 22 and the inside of the barrel 21 by pressure. The flow rate is, e.g., 2cc/min. The barrel 21 can be displaced in the vertical direction in the inside of the through hole 42 extending to the vertical direction formed in the body 34 of the endoscope 33 and the barrel 21 will be projected as shown by the reference number 43 when the light coagulation is carried out so that the end face 24a of the contact member 24 is in contact with the position to be coagulated in the morbid part 38. The area to be treated with the light coagulation is illuminated by 35a of an optical fiber for lighting 35. The movement during the treatment can be observed at the outside by using the display means 39 with the optical fiber for image transmission 36. The body 34 of the endoscope 33 is composed of a flexible material, e.g., gum.

The space 20 is supplied with cooling water and the optical fiber 22 in the state of leading the laser light will be in contact with the morbid part 38 at the end face 24a of the contact member 24 so as to carry out the light coagulation. The end face 29a of the core layer 29 of the optical fiber 22 is at the position far from the morbid part 38 in the distance of ℓ1 when the end face 24a of the contact member 24 is in contact with the morbid part 38. Therefore, the morbid part 38 and blood will not adhere to the end face 29a and the end face 29a is prevented from being heated to an abnormally high temperature. The optical fiber 22 is always in contact with cooling water and therefore cooling of the optical fiber 22 is surely maintained. Consequently, there are obtained some benefits such as that the life of the optical fiber 22 can be extended, laser light of a large output can be used for treatment and the treatment can be carried out at a low cost. The end face 29a of the core layer 29 of the optical fiber 22 is a plane vertical to the axis of the optical fiber 22 so that it can make the laser light distribute the luminous intensity uniform. Therefore, the light coagulation state of the morbid part 38 by laser light is easily conjectured so that operation can be carried out smooth. The external form of the light illuminated from the end face 29a of the core layer 29 of the optical fiber 22 is shown with reference number 44 and the connecting member 23 is in the size not being an obstacle to illumination of the laser light. The inside of the contact member 24 has a good reflection ratio as noted above so that the morbid part 38 is effectively illuminated by the laser light. The laser light from the optical fiber 22 certainly illuminates the morbid part 38 because the cooling water is transparent.

According to the experiment of the inventors of this invention, the light coagulation of 2.2 mmφ and 1.6 mm in depth occurred at the morbid part 38 in the stomach wall when laser light was generated for 12 second from the laser light source 40 having an output of 20 w. Other transparent liquid may be used instead of cooling water. The cooling liquid such as cooling water do not present any problem to the treated patient such as pains by the cooling liquid since the amount of the cooling liquid is a little. Therefore, smooth operation is ensured within the limited treating period and the operation is carried out with safety.

The contact member 24 is convenient from a sanitary point of view since it is screwed in the connecting member 23 and can be screwed out and changed.

The contact member 24 may be exchanged to the new one when it gets damaged, worn away or stained so that the maintenance is easy and it is convenient for sanitary reasons. Additionally, it is possible to remove the connecting member 23 from the barrel 21 and exchange it. The outer faces of the contact member 24 and the flange part 27 of the connecting member 23 may be coated with Teflon (Trade Mark), by which they can be easily wiped out when tissues such as skin adhere to the outer faces. Thus, the contact member 24 and the connecting member 23 can be kept clean.

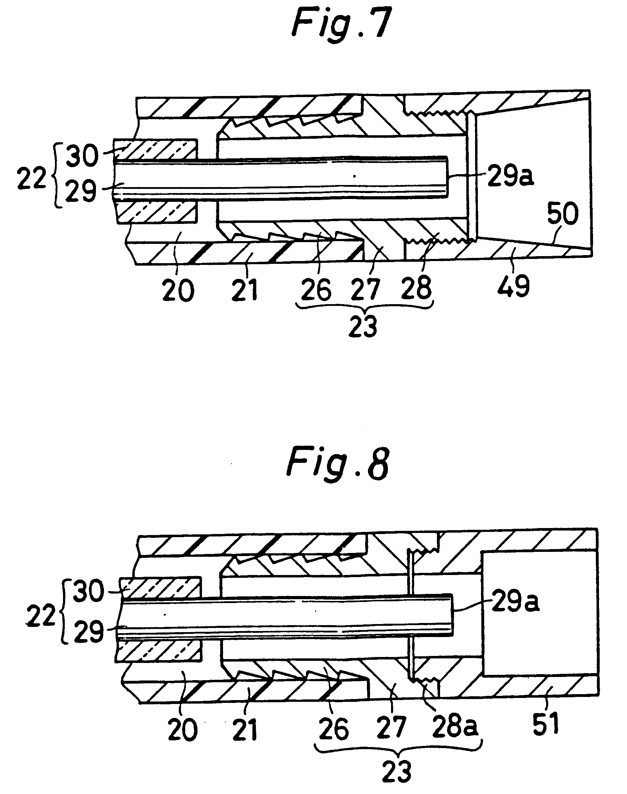

Fig. 7 is a cross-sectional view of other example of this invention, which is similar to the examples shown in Figs. 3 and 4. In this example, the inside 50 of the contact member 49 is formed to be a hollow truncated cone, of which part having larger diameter is put towards the right of Fig. 7, namely the end side.

Fig. 8 is furthermore a cross-sectional view of other example. In this example, a male screw is formed at the starting portion of the contact member 51 and the connecting member 23 has a female screw 28a so that the fixing is performed each other. Additionally, it is possible to remove the connecting member 23 from the barrel 21 and exchange it.

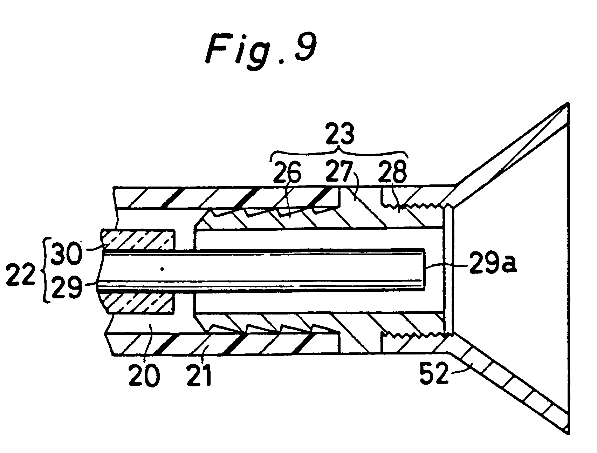

Fig. 9 is a cross-sectional view of other example. In this example, the contact member 52 is formed to be trumpet shaped of which part having larger diameter is put toward the end side. The trumpet shaped contact member 52 may be removed from the connecting member and exchanged. It is possible to remove the connecting member 23 from the barrel 21 and exchange it.

## Claims

1. An apparatus for treatment of portions of a human body with laser light, comprising an optical fiber (29) and a laser light source (40) for generating laser light and for leading the light to the optical fiber (29), a flexible barrel (21) in which the optical fibre (29) is inserted such that a space (20) exists between the outside of the optical fibre (29) and the inside of the barrel (21), means (41) for supplying cooling liquid into the space (20) between the outside of the optical fibre (29) and the inside of the barrel (21), an end portion (29a) of the optical fibre (29) being positioned inner to an end portion (24a) of the barrel (21) in axial alignment therewith, the end portion of the barrel (21) comprising a cylindrical portion (23,24) made of metal, characterized in that in an apparatus for light coagulation of a morbid part, the end portion of the barrel (21) comprises a cylindrical metal connecting member (23) having a connecting portion (26), a flange part (27) and a threaded part (28), the connecting portion (26) being cut such as to taper so as to comprise portions of truncated cones each of said portions having a larger diameter at the side of the flange part (27) of the connecting member (23), the connecting portion (26) being inserted into the outer end of the barrel (21), the flange part (27) being adjacent to the connecting portion (26) in axial direction and projecting in radial direction and contacting an end face (21a) of the barrel (21), said threaded part (28) being at the side of the flange part (27) opposite to the connecting part (26), and a cylindrical metal contact member (24,49,52) being threaded (25) such as to being coupled with the threaded part (28) of the connecting member (23), the end portion (29a) of the optical fibre (29) extending into, but not beyond the connecting member (23).

2. Apparatus according to claim 1, characterized in that the cylindrical metal contact member (52, Fig. 11) is trumpet-shaped, the part having the larger diameter being positioned at the end side.

## Patentansprüche

1. Eine Einrichtung für die Behandlung von Teilen eines menschlichen Körpers mit Laserlicht, mit einer Lichtleitfaser (29) und einer Laserlichtquelle (40) für die Erzeugung von Laserlicht und für die Leitung des Lichts an die Lichtleitfaser (29), einem flexiblen Rohr (21), in das die Lichtleitfaser (29) eingeschoben ist, derart, daß zwischen der Außenseite der Lichtleitfaser (29) und der Innenseite des Rohrs (21) ein Raum (20) vorhanden ist, einer Einrichtung (41) für die Lieferung von Kühlflüssigkeit in den Raum (20) zwischen der Außenseite der Lichtleitfaser (29) und der Innenseite des Rohrs (21, wobei ein Endabschnitt (29a) der Lichtleitfaser (29) gegenüber dem Endabschnitt (24a) des Rohrs (21) zurückgesetzt und zu diesem axial ausgerichtet ist, wobei der Endabschnitt des Rohrs (21) einen aus Metall hergestellten zylindrischen Abschnitt (23, 24) umfaßt, dadurch gekennzeichnet, daß in einer Einrichtung für die Lichtkoagulation eines morbiden Teils der Endabschnitt des Rohrs (21) versehen ist mit einem zylindrischen, metallischen Verbindungselement (23), das einen Verbindungsabschnitt (26), einen Flanschteil (27) und einen Gewindeteil (28) aufweist, wobei der Verbindungsabschnitt (26) kegelstumpfförmig geschnitten ist, derart, daß er Kegelstumpfabschnitte aufweist, von denen jeder auf seiten des Flanschteils (27) des Verbindungselements (23) einen größeren Durchmesser besitzt, wobei der Verbindungsabschnitt (26) in das äußere Ende des Rohrs (21) eingeschoben ist, wobei der Flanschteil (27) in axialer Richtung an den Verbindungsabschnitt (26) angrenzt und in radialer Richtung vorsteht und eine Stirnfläche (21a) des Rohrs (21) berührt, und wobei sich der Gewindeteil (28) auf derjenigen Seite des Flanschteils (27) befindet, die vom Verbindungsabschnitt (26) abgewandt ist, und einem zylindrischen, metallischen Kontaktelement (24, 49, 52), das mit einem Gewinde (25) versehen ist, derart, daß es mit dem Gewindeteil (28) des Verbindungselements (23) verbunden werden kann, wobei sich der Endabschnitt (29a) der Lichtleitfaser (29) im das Verbindungselement (23), jedoch nicht über dieses hinaus erstreckt.

2. Einrichtung gemäß Anspruch 1, dadurch gekennzeichnet, daß das zylindrische, metallische Kontaktelement (52, Fig. 11) trichterförmig ist, wobei sich der Teil mit größerem Durchmesser am Ende befindet.

## Revendications

1. Appareil de traitement de parties du corps humain à la lumière laser, comprenant une fibre optique (29) et une source de lumière laser (40) pour la production de lumière laser et la conduite de celle-ci à la fibre optique (29), un fût souple (21) dans lequel la fibre optique (29) est placée de façon qu'un espace (20) existe entre l'extérieur de la fibre optique (29) et l'intérieur du fût (21), un moyen (41) d'envoi de liquide de refroidissement dans l'espace (20) entre l'extérieur de la fibre optique (29) et l'intérieur du fût (21), une partie d'extrémité (29a) de la fibre optique (29) étant placée à l'intérieur d'une partie d'extrémité (24a) du fût (21) en alignement axial avec celle-ci, la partie d'extrémité du fût (21) comprenant une partie cylindrique (23, 24) en métal, caractérisé par le fait que dans un appareil de photocoagulation d'une partie malade, la partie d'extrémité du fût (21) comprend un élément métallique cylindrique de jonction (23) ayant une partie de jonction (26), un collet (27) et une partie filetée (28), la partie de jonction (26) étant taillée de façon à comprendre des troncs de cône ayant un plus grand diamètre du côté du collet (27) de l'élément de jonction (23), la partie de jonction (26) étant introduite dans l'extrémité extérieure du fût (21), le collet (27) étant contigu à la partie de jonction (26) dans la direction axiale et saillant radialement et étant en contact avec une face d'extrémité (21a) du fût (21), la partie filetée (28) étant sur le côté du collet (27) opposé à la partie de jonction (26), et un élément métallique cylindrique de contact (24, 49, 52) étant taraudé (25) pour être accouplé à la partie filetée (28) de jonction (23), la partie d'extrémité (29a) de la fibre optique (29) entrant dans l'élément de jonction (23), mais ne s'étendant pas au-delà de celui-ci.

2. Appareil selon la revendication 1, caractérisé par le fait que l'élément métallique cylindrique de contact (52, figure 9) est en forme de trompette, sa partie de plus grand diamètre étant située du côté de l'extrémité.
